# EUROPEAN PATENT APPLICATION

(11) **EP 1 270 038 A1**
(43) Date of publication of application: **02.01.2003**
(21) Application number: 01830407.1
(22) Date of filing: 19.06.2001
(51) Int. Cl.: A61M 25/00

(54) **Two-lumen suction catheter for distal protection in a percutaneous intervention**

(71) Applicant: N.G.C. Medical S.p.A., 22060 Novedrate (CO) (IT)
(72) Inventor: Greco, Francesco, c/o N.G.C. Medical S.p.A., 22060 Novedrate (CO) (IT); Silva, Pedro, c/o N.G.C. Medical S.p.A., 22060 Novedrate (CO) (IT)
(74) Representative: Ferraiolo, Rossana

(57) **Abstract**

A the catheter distal tapered part is provided with at least one drop shaped aperture (7) in the second lumen, the aperture having its point distal and the catheter ending with an atraumatic tip (9) to cause turbulence (T) in the blood that flows in the artery region defined between said aperture and said atraumatic tip, said aperture, atraumatic tip and turbulence together causing the sucking into said aperture of those embolic particles that should be released in the suction phase (Fig 2).

## Description

This present invention relates to a two-lumen catheter for distal protection in a percutaneous coronary or peripheral intervention and concerns in general the cardiovascular field and, in particular, the field of coronary and/or peripheral revascularization.

A percutaneous revascularization intervention implies risk of distal embolization particularly in presence of heavy atero-trombic component in the region concerned.

The medical-cardiologic community realized that said risk is important especially when it is associated with adverse peri-procedural events and likely with a bad long-term prognosis. Due to the above reasons, research and bioengineering industry developped and put on the market a number of protection systems substantially consisting of filters or occlusive devices capable of preventing migration of atero debris during a intervention.

Known filters and systems imply certain drawbacks: manipulation is difficult; time for preparing and positioning such means is notable; certain complications like spasms and intimal dissection may set in which do not assure a total protection from embolizations during the positioning of the means.

This invented catheter obviates said drawbacks since it reduces the athero-trombotic burden in the artery concerned and simultaneously provides for a distal protection in the suction phase; in turn, said advantages make easy the manipulation and reduce the time of intervention and the probability of complications. Finally, a intervention which uses this catheter proves to be less expensive than the other known.

The invented catheter is a two-lumen catheter of the type that can be positioned into the artery by using a conventional angioplastic guide (for instance, a steel wire guide of 0.014") passing through its first lumen of small section and wherein the proximal end of the higher section second lumen is provided with connecting means allowing use of a conventional syringe for sucking in potential embolic material, the catheter being characterized in that comprises a the distal tapered part provided with at least one aperture, preferably drop shaped, in the second lumen, the drop shaped aperture having its point distal and the catheter ending with an atraumatic tip, substantially in the form of a ball, to cause turbulence in the blood flowing in the artery region defined between said aperture and said atraumatic tip, said aperture, atraumatic tip and turbulence together causing the sucking into said aperture of those embolic particles that should be released in the suction phase.

The invention will be now described by way of example and with reference to the drawing in which
Fig. 1 is a general perspetive view,
Fig. 2 is a part perspective view and
Fig. 3 is a part plant view.

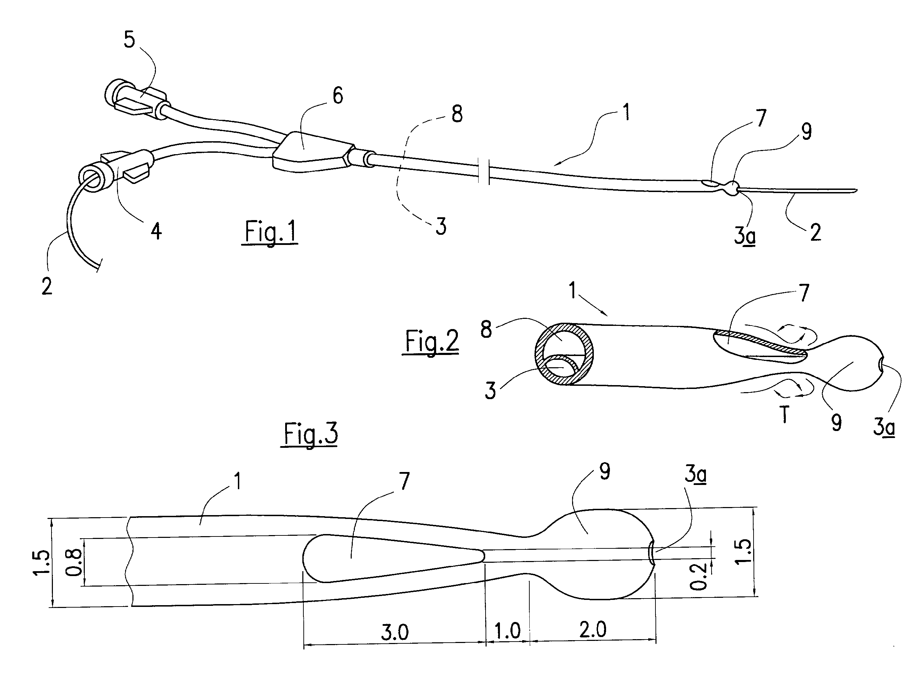

Fig. 1 shows a two-lumen catheter 1 with an angioplastic guide 2 going along the small section lumen 3 from a first proximal connector 4 to the hole 3a on the distal end ; a second proximal connector 5 is provided for applying a syringe, not shown, for use in the sucking phase; ducts connected with first and second proximal connectors 4, 5 converge in the connector 6 in such a way that the first duct is connected with the small section lumen 3 and the second duct is connected with the higher section lumen 8. On the distal end of the catheter a drop shaped aperture 7 in the lumen 8 and an atraumatic tip 9 are visible.

Fig. 2 details the catheter distal end ; on the left side of the figure, lumina 3, 8 are shown, respectively of small and higher section ; the right side shows the drop shaped aperture 7 made in the distal tapered part, between the end of cylindric part and the beginning of the atraumatic tip 9. The drop shaped aperture 7 has the large round edge upstream and the point downstream, adjacent to the atraumatic tip. Once the catheter is set in an artery, turbulence T is caused in the blood flow, which turbulence helps in trapping possible embolic particles and, in the suction phase, makes them to enter the aperture 7.

Fig. 3 shows an example of adapted sizes in the distal end of a catheter 1; the outer diameter is 1.5 mm and the tapered end comprises the drop shaped aperture 7 which is extended parallel with the longitudinal central line of the catheter and is 3.0 mm long; the upstream round edge has 0.4 mm radius and the downstream round edge has 0.1 mm radius; the atraumatic tip 9 is 2.0 mm long and 1.5 mm in diameter.

## Claims

1. A two-lumen catheter (I) for distal protection in a percutaneous coronary or peripheral intervention of the type that can be positioned into an artery by using a conventional angioplastic guide (2) passing through its first lumen of small section (3) and wherein the proximal end of the higher section second lumen (8) is provided with connecting means (5) allowing use of a conventional syringe for sucking in potential embolic material, the catheter being **characterized in that** comprises a distal tapered part provided with at least one aperture (7) in the second lumen (8), the catheter ending with an atraumatic tip (9) to cause turbulence (T) in the blood that flows in the artery region defined between said aperture and said atraumatic tip, said aperture, atraumatic tip and turbulence together causing sucking into said aperture of those embolic particles that should be released in the suction phase.

2. A two-lumen catheter (I) according to claim 1 **characterized in that** the aperture (7) is drop shaped with its point downstream and extends parallel with the longitudinal center line of the catheter.

3. A two-lumen catheter (1) according to claims 1 and 2 **characterized in that** the drop shaped aperture (7) is about 3.0 mm long, the upstream round edge has about 0.4 mm radius and the downstream round edge has about 0.1 mm radius, the atraumatic tip (9) being about 2.0 mm long and about 1.5 mm in diameter.
